# EUROPÄISCHE PATENTSCHRIFT

(11) **EP 1 318 131 B1**
(45) Veröffentlichungstag und Bekanntmachung des Hinweises auf die Patenterteilung: **06.07.2005**
(21) Anmeldenummer: 02027070.8
(22) Anmeldetag: 03.12.2002
(51) Int. Cl.: C07C 29/17, C07C 31/125

(54) **Verfahren zur Herstellung von Tetrahydrogeraniol**
Process for the preparation of tetrahydrogeraniol
Procédé pour la préparation de tétrahydrogéraniol

(30) Priorität: 07.12.2001 DE 10160142
(43) Veröffentlichungstag der Anmeldung: 11.06.2003
(73) Patentinhaber: BASF AKTIENGESELLSCHAFT, 67056 Ludwigshafen (DE)
(72) Erfinder: Funke, Frank, Dr., 68161 Mannheim (DE); Gerlach, Till, Dr., 67071 Ludwigshafen (DE); Ebel, Klaus, Dr., 68623 Lampertheim (DE); Unverricht, Signe, Dr., 68169 Mannheim (DE); Haese, Frank, Dr., 67245 Lambsheim (DE); Burkart, Kirsten, 67069 Ludwigshafen (DE); Göbbel, Hans-Georg, Dr., 67169 Kallstadt (DE)

(56) Entgegenhaltungen:
- GB-A- 989 262
- SINGH U.K. ET AL.: "Liquid-Phase Hydrogenation of Citral over Pt/SiO2 Catalysts" JOURNAL OF CATALYSIS, Bd. 191, 2000, Seiten 181-191, XP002252246
- DATABASE WPI Derwent Publications Ltd., London, GB; AN 1980-87947C XP002252248 "Production of citronellol for use as scent in perfumery by hydrogenation of citral in aqueous alcohol in presence of nickel-chromium catalyst with addition of soda." & SU 729 183 A (AS KAZA ORGAN CATAL), 28. April 1980 (1980-04-28)
- DATABASE WPI Derwent Publications Ltd., London, GB; AN 1977-65638Y XP002252249 "Citronellol preparation by partial hydrogenation of geraniol using catalyst obtained by oxidising triphenyl-phosphine iridium chelate derivatives." & JP 52 091811 A (INST PHYSICAL & CHEM RES), 2. August 1977 (1977-08-02)
- DATABASE WPI Derwent Publications Ltd., London, GB; AN 1985-287121 XP002252250 "Unsaturated alcohol preparation by hydrogenating (poly)isoprenyl but-2-en-1-al derivatives in presence of Raney-nckel and -cobalt" & JP 60 197634 A (KURARAY CO LTD), 7. Oktober 1985 (1985-10-07)
- DATABASE WPI Derwent Publications Ltd., London, GB; AN 1982-21100E XP002252251 "Preparation of citronellol used in perfumes by reducing geraniol with hydrogen in presence of nickel diatomaceous earth catalyst and alkali metal compound" & JP 57 024320 A (TOYOTAMA KORYO KK), 8. Februar 1982 (1982-02-08)
- MATTEOLI U. ET AL.: "Asymmetric hydrogenation by an in situ prepared (S)-BINAP-Ru(II) catalytic system" JOURNAL OF MOLECULAR CATALYSIS A: CHEMICAL, Bd. 140, 1999, Seiten 131-137, XP002252247

## Beschreibung

Die vorliegende Erfindung betrifft ein Verfahren zur Herstellung von Tetrahydrogeraniol, wobei die Destillationsrückstände bzw. die Produktgemische aus der Linalool-, der Citronellal-, Citronellol- oder der Geraniol-/Nerol-Synthese direkt der katalytischen Hydrierung zugeführt werden.

Tetrahydrogeraniol (3,7-Dimethyloktanol) stellt ein wichtiges Zwischenprodukt in der industriellen organischen Synthese dar und wird als Riechstoff oder als Additiv in Seifen und Detergentien verwendet.

Die Herstellung von Tetrahydrogeraniol durch Hydrierung der ungesättigten Vorläufer wie Citral, Citronellol oder Citronellal bzw. Nerol/Geraniol ist lange bekannt. Schon 1912 wurde die Hydrierung aus Citral an Palladium beschrieben (Ipatjew, Chem. Ber., 45, 1912, 3222). An Metallen auf Kalium-Graphit-Trägern wird die Hydrierung von Citral in Savoia, Tagliavini, Trombini, Umani-Ronchi J. Org.Chem., 1981, 46, 5344-5348 beschrieben. An einem Ni/Graphit-Kontakt erhält man Tetrahydrogeraniol in einer Ausbeute von 95 %. 1993 gelang die Hydrierung von Citronellol zu Tetrahydrogeraniol an einem Pd/C-Kontakt in Ausbeuten von 93 %. Die Hydrierung von 3,7-Dimethyloktanal an einem homogenen (NiCl₂(PPh₃)₂) wurde in J. Chem. Soc. Chem. Com. 1995 (Iyer, Varghese (4), 465-466) dokumentiert. Sie gelingt in einer Ausbeute von 57 %.

Die Hydrierung bereits kostspielig hergestellter Vorläufer, die in reiner Form vorliegen, wie Citral, Nerol oder Geraniol ist aus wirtschaftlichen Aspekten nicht sinnvoll. Eine Überhydrierung, z.B. bei der Herstellung von Nerol/Geraniol ist aufgrund der schlechten Abtrennbarkeit von Tetrahydrogeraniol von den ungesättigten Produkten nicht ratsam.

Der vorliegenden Erfindung lag die Aufgabe zugrunde, ein wirtschaftliches Verfahren zur Herstellung von Tetrahydrogeraniol durch Umsetzung bzw. Verwendung von Rückständen, die bei der Herstellung von Linalool, Citronellal, Citronellol bzw. Geraniol/Nerol entstehen, zu entwickeln.

Es sollten Möglichkeiten gefunden werden, die es erlauben, die Hydrierung der Rückstände aus den oben genannten partiellen Hydrierungen bzw. Umlagerungen, insbesondere aus der Linalool-Synthese mit hohem Umsatz, guter Ausbeute, Selektivität und Katalysatorstandzeit, durchzuführen.

Überraschenderweise wurde die Aufgabe erfindungsgemäß gelöst durch ein Verfahren zur Herstellung von Tetrahydrogeraniol, dadurch gekennzeichnet, dass die bei der Citronellol-, Linalool-, Citronellal- oder Geraniol-/Nerol-Synthese anfallenden Produktgemische bzw. Destillationsrückstände direkt der katalytischen Hydrierung zugeführt werden.

Als Einsatzstoff können alle Produktgemische bzw. Destillationsrückstände dienen, die mehr als eine Verbindung der folgenden Grundstruktur der allgemeinen Formel I enthalten 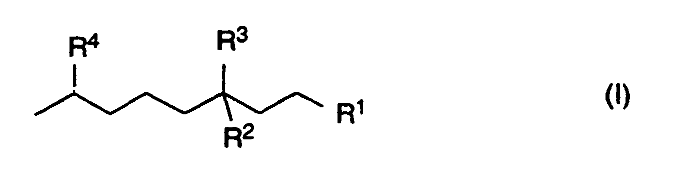 wobei
- R¹: OH, H, CH₃
- R², R³: H, OH, CH₃
- R⁴: H, CH₃ bedeutet,
und ein bis vier Doppelbindungen an beliebiger Stelle im Molekül enthalten sein können.

Bevorzugt enthalten die Produktgemische bzw. Destillationsrückstände mehr als eine Verbindung ausgewählt aus der Gruppe Nerol, Geraniol, Isonerol 2, Citral, Citronellol, 3,7-Dimethyloctanal, Isonerol 1, Citronellal, Linalool, (von oben nach unten, Linalool ist nicht dargestellt, s. Formelschema).

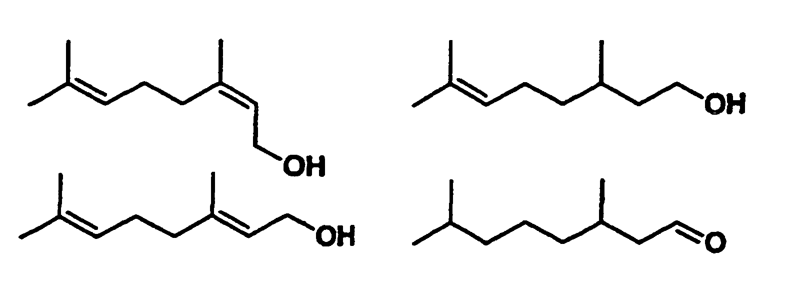

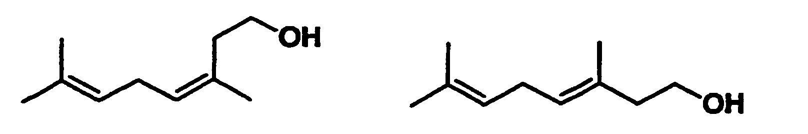

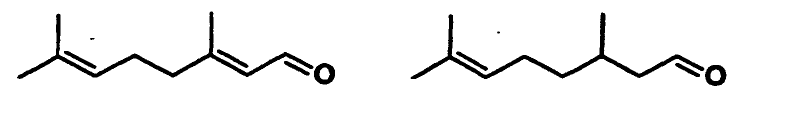

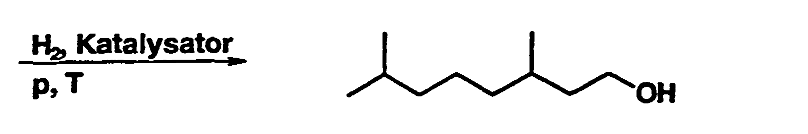

Verwendet man beispielsweise, die aus der Linalool-Synthese erhaltenen Rückstände (Tabellel), so beträgt der Sumpf-Gehalt an Linalool und Geraniol/Nerol des eingesetzten Rückstandes bzw. Produktgemisches in Summe weniger als 30 Gew.-%, bevorzugt weniger als 20 Gew-%, insbesonders bevorzugt weniger als 10 Gew.-%. Der Anteil von Citronellol und iso-Nerol beträgt in Summe mehr als 50 Gew-%, bevorzugt mehr als 80 Gew.-%.

**Tabelle 1**

| Typischer Rückstand aus einer Linalool-Synthese in Gew.-%: | |
|---|---|
| Leichtsieder | 0,10 |
| Tetrahydrolinalool | 0,00 |
| Linalool | 6,58 |
| Citronellol | 31,99 |
| Nerol | 0,57 |
| Citral (cis/trans) | 1,04 |
| Tetrahydrogeraniol | 0,03 |
| iso-Nerol (I+II) | 54,60 |
| andere | 5,05 |

Verwendet man beispielsweise, die aus der Nerol/Geraniol-Synthese erhaltenen Rückstände (Tabelle 2), so beträgt der Sumpf-Gehalt an verwertbaren Produkten (Geraniol und iso-Nerole) knapp 60 %.

**Tabelle 2**

| Typischer Rückstand aus einer Nerol/Geraniol-Synthese in Gew.-%: | |
|---|---|
| Leichtsieder | 1 |
| Geraniol | 45 |
| iso-Nerol (I+II) | 13 |
| Hochsieder | 41 |

Verwendet man beispielsweise, die aus der Citronellal-Synthese erhaltenen Rückstände (Tabelle 3), so beträgt der Sumpf-Gehalt an verwertbaren Produkten knapp 90 %.

**Tabelle 3**

| Typischer Rückstand aus einer Citronellal-Synthese in Gew.-%: | |
|---|---|
| Citral | 44 |
| Citronellal | 30 |
| Citronellol | 15 |
| Hochsieder | 11 |

Verwendet man beispielsweise, die aus der Citronellol-Synthese erhaltenen Rückstände (Tabelle 4), so beträgt der Sumpf-Gehalt an verwertbaren Produkten (Geraniol und iso-Nerole) 95 %.

**Tabelle 4**

| Typischer Rückstand aus einer Citronellol-Synthese in Gew.-%: | |
|---|---|
| Geraniol | 10 |
| Nerol | 5 |
| Citronellol | 75 |
| Dimethyloktanol | 5 |
| Hochsieder | 5 |

Als Hydrierkatalysatoren sind prinzipiell alle Hydrierkatalysatoren geeignet, welche zur Hydrierung olefinischer Doppelbindungen eingesetzt werden können (z.B. Houben Weyl, Methoden der organischen Chemie, Band 4/1c).

Als Hydrierkatalysatoren besonders geeignet sind Katalysatoren, die als katalytisch aktive Bestandteile Elemente, ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, jeweils in metallischer Form (z.B. als Ra-Katalysator, Oxidationsstufe 0) oder in Form von Verbindungen wie z. B. Oxiden, die unter den Verfahrensbedingungen zum entsprechenden Metall reduziert werden, enthalten.

Die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram sind im allgemeinen insgesamt in Mengen von 0,1 bis 80 Gew.-%, bevorzugt 0,1 bis 70 Gew.-%, besonders bevorzugt 0,1 bis 60 Gew.-%, berechnet als Metall in der Oxidationsstufe 0, in der katalytisch aktiven Masse des Katalysators enthalten.

Bevorzugt sind Katalysatoren, die als katalytisch aktive Bestandteile Elemente, ausgewählt aus der Gruppe Kupfer, Silber, Cobalt, Nickel, Ruthenium, Rhodium, Palladium, Platin, Chrom und Molybdän, insbesondere ausgewählt aus der Gruppe, Kupfer, Cobalt, Nickel, Palladium jeweils in metallischer Form (Oxidationsstufe 0) oder in Form von Verbindungen wie z.B. Oxiden, die unter den Verfahrensbedingungen zum entsprechenden Metall reduziert werden, enthalten.

Besonders bevorzugt sind Katalysatoren mit den Aktivkomponenten Co, Ni, Cu Ru, Pd und/oder Pt. Diese können als Vollkontakte oder als Trägerkatalysatoren bzw. aktivierte Metallkatalysatoren (Raney-Katalysatoren) verwendet werden.

Weiterhin enthält die katalytisch aktive Masse dieser bevorzugt eingesetzten Katalysatoren die Trägermaterialien Aluminiumoxid (Al₂O₃), Zirkoniumdioxid (ZrO₂), Titandioxid (TiO₂), Kohlenstoff und/oder sauerstoffhaltige Verbindungen des Siliciums, berechnet als SiO₂, im allgemeinen insgesamt in Mengen von 20 bis 99,9 Gew.-%, bevorzugt 30 bis 99,9 Gew.-%, besonders bevorzugt 40 bis 99,9 Gew.-%.

Unter den Raney-Katalysatoren werden bevorzugt Raney-Katalysatoren wie Ra-Ni. Ra-Co, Ra-Co-Ni-Fe, Ra-Ni-Co-Fe-Cr oder Ra-Ni und Ra-Co mit Dotierungen anderer Übergangsmetalle in wasserfreier oder auch wasserfeuchter oder lösungsmittelfreier Form eingesetzt verwendet.

Die im erfindungsgemäßen Verfahren als Hydrierkatalysator verwendeten Hydrierkatalysatoren können nach den im Stand der Technik beschriebenen Verfahren hergestellt und teilweise auch kommerziell erhalten werden.

Die Hydrierung kann man diskontinuierlich oder kontinuierlich durchführen. Bei größeren Mengen (>500 t/a) ist eine kontinuierlich betriebene Hydrierung ratsam.

Man kann die Umsetzung in einer Suspension oder in einem Festbett durchführen. Bei einer kontinuierlich betrieben Festbettvariante kann in Riesel- oder Sumpffahrweise gearbeitet werden. Denkbar ist auch die Hydrierung in der Gasphase.

Die Suspensionshydrierung kann man diskontinuierlich, in der Regel in der Flüssigphase durchführen.

Für alle Varianten wählt man Temperaturen zwischen 20-250°C, bevorzugt 30-200°C, besonders bevorzugt 50-180°C.

Den Druck wählt man im allgemeinen im Bereich von 1-250 bar, bevorzugt 5-200 bar, besonders bevorzugt 10-100 bar.

Die Hydrierung im erfindungsgemäßen Verfahren kann sowohl ohne Lösungsmittel als auch in Gegenwart eines Lösungsmittels, beispielsweise Alkoholen wie Methanol, Ethanol, Propanol, Butanol, aliphatischen oder aromatischen Kohlenwasserstoffen wie Toluol, Xylol, Cyclohexan, Ether wie THF, Dioxan, Methyl-tert-butylether durchgeführt werden.

Bevorzugt findet die Hydrierung jedoch ohne Lösungsmittel statt.

Die folgenden Beispiele sollen das erfindungsgemäße Verfahren näher erläutern, ohne es jedoch darauf zu beschränken.

### Linalool-Synthese:

Im Sumpf der Kolonne wird mit homogen gelöstem Katalysator Geraniol/Nerol zu Linalool isomerisiert und das gebildete Linalool kontinuierlich abdestilliert, so dass sich im Sumpf eine stationäre Linalool-Konzentration von 3-8 Gew.-% einstellt. Im gleichen Maße wie Linalool abdestilliert, wird Geraniol/Nerol in den Sumpf kontinuierlich nachdosiert. Dabei werden Hochsieder sowie Citronellol und Isonerole im Sumpf aufgepegelt. Bei Erreichen eines Anteils von z.B.: 20 Gew.-% Hochsieder, 16 Gew.-% Citronellol und 24 Gew.-% Isonerol I+II wird die Geraniol/Nerol -Zufuhr abgestellt und das Rest- Geraniol/Nerol im Sumpf weiter isomerisierend als Linalool abdestilliert. Es wird solange weiter isomerisiert, bis der Sumpf-Gehalt an Linalool, Geraniol/Nerol Summe 10 Gew-% oder weniger beträgt.

Sodann wird der Druck von 135 mbar kontinuierlich auf 5 mbar (nur Bsp) abgesenkt und Citronellol und Isonerole, gemeinsam mit restlichem Linalool abdestilliert. Zusammensetzung: 35 % Lin, 31 % Citronellol, 34 % Isonerol I+II. Der Rückstand (siehe Bespiele 1-4) wird dann erfindungsgemäß dort oder anderer Stelle hydriert.

### Beispiel 1:

In einem 270 ml-Autoklav mit Rührer und Katalysatorkorb 20 ml Katalysator 1 (Herstellung (0,47 % Pd auf γ-Aluminiumoxid: Der Katalysator wurde hergestellt durch Tränkung von γ-Aluminiumoxidsträngen (Durchmesser der Stränge: 4 mm) mit einer wässrigen Palladiumnitratlösung. Die Pd-Konzentration in der Tränklösung wurde so eingestellt, dass die Stränge mit etwa 95 % ihrer Aufnahmekapazität für Wasser getränkt wurden und ein Edelmetallgehalt von 0,47 Gew.-% bezogen auf das Gesamtgewicht des fertigen Katalysators resultiert. Die getränkten Stränge wurden anschließend für 12 h bei 120°C getrocknet und für 6 h bei 300°C calciniert.) gegeben. Dieser wurde drucklos mit 10 Nl/h Wasserstoff auf 280°C erhitzt und anschließend 3h bei 40 bar Wasserstoffdruck gehalten. Dann wurden bei RT 50 ml des Gemisches 1 in den Reaktor gegeben. Bei einem Druck von 40 bar wurde die Reaktion bei 100°C durchgeführt

| Analytik: 30 m DBWAX 0,32 mm 0,25 µm 80°C - 3°C/min - 230°C - 20min | | | |
|---|---|---|---|
| | Einsatzgemisch | Produktgemisch (3h) | Produktgemisch (5h) |
| | | | |
| Leichtsieder | 0,00 | 1,43 | 1,52 |
| Tetrahydrolinalool | 0,00 | 6,69 | 6,69 |
| Linalool | 6,58 | 0 | 0 |
| Citronellol | 31,99 | 0 | 0 |
| Nerol | 0,57 | 0,34 | 0,10 |
| Citral (cis/ trans) | 1,08 | 0,00 | 0 |
| Tetrahydrogeraniol | 0,03 | 85,27 | 85,63 |
| iso-Nerol (I+II) | 54,61 | 0,30 | 0,15 |
| Andere | 4,60 | 5,94 | 5,95 |

Der Umsatz (bezogen auf Komponenten, die zu Dimethyloktanol hydriert werden können), betrug 99,6 %. Die Selektivität betrug 98,3 %.

### Beispiel 2

In einem 270 ml-Autoklav mit Rührer und Katalysatorkorb 20 ml Katalysator 2 (NiO 21,5 %, Na₂O 0,35 %, CuO 7,3 %, Mn₃O₄ 2,0 % und H₃PO₄ 1,2 % bei 190°C reduziert/passiviert) gegeben. Dieser wurde drucklos mit 10 Nl/h Wasserstoff auf 280°C erhitzt und anschließend 3 h bei 40 bar Wasserstoffdruck gehalten. Dann wurden bei RT 50 ml des Gemisches 1 in den Reaktor gegeben. Bei einem Druck von 40 bar wurde die Reaktion bei 100°C durchgeführt

| Analytik: 30 m DBWAX 0,32 mm 0,25 µm 80°C - 3°C/min - 230°C - 20min | | | |
|---|---|---|---|
| | Einsatzgemisch | Produktgemisch (3h) | Produktgemisch (6h) |
| | | | |
| Leichtsieder | 0,10 | 0,70 | 1,10 |
| Tetrahydrolinalool | 0,00 | 5,85 | 6,50 |
| Linalool | 6,58 | 0 | 0,00 |
| Citronellol | 31,99 | 4,60 | 0,30 |
| Nerol | 0,57 | 0,70 | 0,14 |
| Citrat (cis/trans) | 1,04 | 0 | 0,12 |
| Tetrahydrogeraniol | 0,03 | 79 | 86,3 |
| iso-Nerol (I+II) | 54,60 | 0 | 0,2 |
| Andere | 5,05 | 8 | 5,40 |

Der Umsatz (bezogen auf Komponenten, die zu Dimethyloktanol hydriert werden können), betrug 98,9 %. Die Selektivität betrug 98,7 %.

### Beispiel 3

In einem 270 ml-Autoklav mit Rührer und Katalysatorkorb 20 ml Katalysator 3 (Katalysator A aus EP 0963975 A1) gegeben. Dieser wurde drucklos mit 10 Nl/h Wasserstoff auf 280°C erhitzt und anschließend 3 h bei 40 bar Wasserstoffdruck gehalten. Dann wurden bei RT 50 ml des Gemisches 1 in den Reaktor gegeben. Bei einem Druck von 40 bar wurde die Reaktion bei 180°C durchgeführt

| Analytik: 30 m DBWAX 0,32 mm 0,25 µm 80°C - 3°C/min - 230°C - 20min | | | |
|---|---|---|---|
| | Einsatzgemisch | Produktgemisch (2h) | Produktgemisch (4h) |
| | | | |
| Leichtsieder | 0,10 | 4,28 | 7,32 |
| Tetrahydrolinalool | 0,00 | 6,41 | 6,44 |
| Linalool | 6,58 | 0,00 | 0,00 |
| Citronellol | 31,99 | 0,46 | 0,18 |
| Nerol | 0,57 | 0,3 | 0,10 |
| Citral (cis/trans) | 1,04 | 0,22 | 0,09 |
| Tetrahydrogeraniol | 0,03 | 81,75 | 78,03 |
| iso-Nerol (I+II) | 54,60 | 0,28 | 0,15 |
| Andere | 5,05 | 6,3 | 7,69 |

Der Umsatz (bezogen auf Komponenten, die zu Dimethyloktanol hydriert werden können), betrug 99,4 %. Die Selektivität betrug 91,4 %.

### Beispiel 4

In einem 270 ml-Autoklav mit Rührer und Katalysatorkorb 20 ml Katalysator (Kat A, Beispiel 3) gegeben. Dieser wurde drucklos mit 10 Nl/h Wasserstoff auf 280°C erhitzt und anschließend 3 h bei 40 bar Wasserstoffdruck gehalten. Dann wurden bei RT 50 ml des Gemisches 1 in den Reaktor gegeben. Bei einem Druck von 40 bar wurde die Reaktion bei 160°C durchgeführt

| Analytik: 30 m DBWAX 0,32 mm 0,25 µm 80°C - 3°C/min - 230°C - 20min | | | |
|---|---|---|---|
| | Einsatzgemisch | Produktgemisch (2h) | Produktgemisch (4h) |
| | | | |
| Leichtsieder | 0,10 | 2,87 | 3,49 |
| Tetrahydrolinalool | 0,00 | 6,30 | 6,24 |
| Linalool | 6,58 | 0,00 | 0,00 |
| Citronellol | 31,99 | 0,08 | 0,08 |
| Nerol | 0,57 | 0,36 | 0,18 |
| Citral (cis/trans) | 1,04 | 0,27 | 0,09 |
| Tetrahydrogeraniol | 0,03 | 82,17 | 80,55 |
| iso-Nerol (I+II) | 54,60 | 0,28 | 0,15 |
| Andere | 5,05 | | |

Der Umsatz (bezogen auf Komponenten, die zu Dimethyloktanol hydriert werden können), betrug 99,4 %. Die Selektivität betrug 95,9 %.

### Beispiel 5

In einem 50 ml-Glas-Autoklav wurden 0,1 g ml Katalysator gegeben. Der Autoklav wurde dreimal mit 5 bar Argon und anschließend 3* mit 10 bar H2 gespült, nachdem Edukte (10 g) vorgelegt werden. Die Reaktionsbedingungen sind der Tabelle zu entnehmen.

### Bedingungen:

| Versuchs-Nr. | Kat | Temperatur In °C | Druck | H2-Verbrauch | Laufzeit |
|---|---|---|---|---|---|
| | | | In bar | In bar | In h |
| 1 | 5% Pd/C (Heraeus; 46 % Wasser; Typ K-0293) | 85 | 20 | 8,1 | 2 |
| 2 | 5% Pd/C (s.o.) | 125 | 20 | 25 | 2 |
| 3 | 10 % Pd/C (Degussa; Typ E10 N/D) | 125 | 20 | 20,5 | 2 |
| 4 | PtO2 * H20 (Fluka) | 125 | 20 | 3 | 2 |
| 5 | Pd/C H0-50 (DE 2936362) | 125 | 20 | 35 | 3 |
| 6 | Pd/C H0-50 | 130 | 20 | 81,5 | 5 |
| 7 | Pd/C H0-50 | 125 | 60 | Nicht ablesbar | 4 |

| Versuchs-Nr. | | 1 | 2 | 3 | 4 | 5 | 6 | 7 |
|---|---|---|---|---|---|---|---|---|
| Edukt | | | | | | | | |
| Leichtsieder | 0,82 | 3,10 | 19,41 | 18,92 | 1,0 | 11,55 | 8,76 | 9,94 |
| Linalool | 1,43 | 1,23 | 0,76 | 0,79 | 1,38 | 1,05 | - | - |
| Mittelsieder | 1,51 | 10,10 | 18,58 | 16,80 | 2,62 | 18,51 | - | - |
| Citronellol | 57,35 | 54,63 | 37,76 | 35,84 | 57,05 | 36,88 | - | - |
| Nerol | 0,14 | 0,52 | - | - | 0,13 | - | - | - |
| Iso-Nerol I | 28,56 | 17,43 | 0,08 | 0,16 | 25,50 | 0,12 | - | - |
| Iso-Nerol II | 10,08 | 7,14 | 0,03 | 0,09 | 9,20 | 0,04 | - | - |
| Geraniol | 0,02 | 1,19 | 0,02 | 0,02 | 0,08 | 0,03 | - | - |
| Hochsieder | 0,06 | 0,02 | 2,50 | 3,48 | 0,56 | 1,05 | 1,00 | 0,49 |
| Tetrahydrogeraniol | - | 4,62 | 20,57 | 23,87 | 1,93 | 30,72 | 90,24 | 89,56 |

### Alle Analysen in Fl.-%.

Von Probe 895 wurde GC/MS durchgeführt. Tetrahydrogeraniol 89,56 % und 7,80 % Tetrahydrolinalool sowie Hauptleichtsiederpeak (2,6 Dimethyloctan) mit 1,74 % werden identifiziert.

### Beispiel 6

In einem 2,5 1 Autoklaven werden 680 g Edukt Mischung aus 36 % Citronellol, 58 % Isonerol I+II, 4 % Linalool, und 4 % sonstigen Verbindungen mit 6,8 g Pd/C (5 % Pd) vorgelegt und bei 125°C bei 60 bar H2 etwa 5,5 h hydriert. Der abgekühlte Reaktionsaustrag enthielt nach Filtration vom Katalysator 92,2 % Tetrahydrogeraniol und 4 % Tetrahydrolinalool.

Der filtrierte Rohaustrag wurde bei 103-112°C Sumpftemperatur und 9-10 mbar bei einem Rücklaufverhältnis von 25:1 bis 5:1 destilliert. Die Fraktionierung ergab 557 g Tetrahydrogeraniol mit einem Gehalt > 99 % von guter Riechstoffqualität mit (Sdp.: 98°C bei 9-10 mbar). Im Vor- und im Destillationsrückstand war noch weiteres Tetrahydrogeraniol enthalten.

### Beispiel 7

In einem 2,5 1 Autoklaven werden 680 g Edukt Mischung aus 38 % Citronellol, 56 % Isonerol I+II, 5 % Linalool, und 3 % sonstigen Verbindungen mit 5 g Ra-Ni (Degussa) vorgelegt und bei 100°C bei 90 bar H2 etwa 12 h hydriert. Der abgekühlte Reaktionsaustrag enthielt nach Filtration vom Katalysator 93,2 % Tetrahydrogeraniol und 5 % Tetrahydrolinalool.

## Patentansprüche

1. Verfahren zur Herstellung von Tetrahydrogeraniol, **dadurch gekennzeichnet, dass** die bei der Linalool-, Citronellal-, Citronellol- oder Geraniol-/Nerol-Synthese anfallenden Produktgemische bzw. Destillationsrückstände direkt der katalytischen Hydrierung zugeführt werden.

2. Verfahren gemäß Anspruch 1, **dadurch gekennzeichnet, dass** die Produktgemische bzw. Destillationsrückstände mehr als eine Verbindung der allgemeinen Formel (I) enthalten 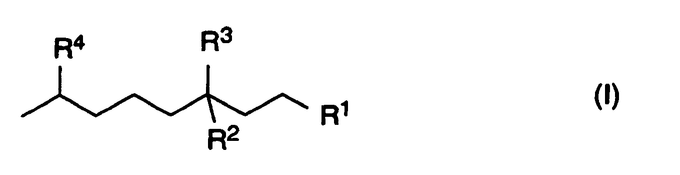 wobei
R¹ OH, H, CH₃
R², R³ H, OH, CH₃
R⁴ H, CH₃ bedeutet,
und ein bis vier Doppelbindungen an beliebiger Stelle im Molekül enthalten sein können.

3. Verfahren gemäß einem der Ansprüche 1 oder 2, **dadurch gekennzeichnet dass** die Produktgemische bzw. Destillationsrückstände mehr als eine Verbindung ausgewählt aus der Gruppe Nerol, Geraniol, Isonerol 2, Citral, Citronellol, 3,7-Dimethyloctanal, Isonerol 1, Citronellal, Linalool enthalten.

4. Verfahren gemäß einem der Ansprüche 1 bis 4, **dadurch gekennzeichnet, dass** der Destillationsrückstand bzw. das Produktgemisch aus der Linalool-Synthese verwendet wird.

5. Verfahren gemäß Anspruch 4, **dadurch gekennzeichnet, dass** der Sumpf-Gehalt an Linalool und Geraniol/Nerol in Summe kleiner als 30 Gew-% beträgt.

6. Verfahren gemäß einem der Ansprüche 4 oder 5, **dadurch gekennzeichnet, dass** das Produktgemisch einen Anteil von größer als 50 Gew.-% Citronellol und Isonerol in Summe enthält.

7. Verfahren gemäß einem der Ansprüche 1 bis 6, **dadurch gekennzeichnet, dass** die Hydrierung an Metallkatalysatoren erfolgt.

8. Verfahren gemäß Anspruch 7, **dadurch gekennzeichnet, dass** die Metallkatalysatoren ausgewählt sind aus der Metalle der Gruppe VIIIA des Periodensystems.

9. Verfahren gemäß Anspruch 7 oder 8, **dadurch gekennzeichnet, dass** die Metallkatalysatoren Pd, Co oder Ni enthalten.

10. Verfahren gemäß einem der Ansprüche 7 bis 9, **dadurch gekennzeichnet, dass** die Hydrierung an Raney-Nickel durchgeführt wird.

11. Verwendung von Produktgemischen bzw. den Destillationsrückständen aus der Linalool-, Citronellal, Citronellol oder Geraniol-/Nerol-Synthese zur Herstellung von Tetrahydrogeraniol.

12. Verwendung von Produktgemischen bzw. den Destillationsrückständen aus der Linalool-Synthese zur Herstellung von Tetrahydrogeraniol an Ra-Katalysatoren.

## Claims

1. A process for the preparation of tetrahydrogeraniol, wherein the product mixtures and distillation residues resulting from linalool, citronellal, citronellol or geraniol/nerol synthesis are fed directly to catalytic hydrogenation.

2. The process as claimed in claim 1, wherein the product mixtures or distillation residues comprise more than one compound of the general formula (I) 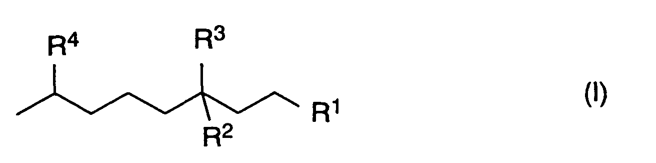 where
R¹ is OH, H, CH₃
R², R³ are H, OH, CH₃
R⁴ is H, CH₃,
and one to four double bonds can be present at any site in the molecule.

3. The process as claimed in claim 1 or 2, wherein the product mixtures or distillation residues comprise more than one compound selected from the group consisting of nerol, geraniol, isonerol 2, citral, citronellol, 3,7-dimethyloctanal, isonerol 1, citronellal and linalool.

4. The process as claimed in any of claims 1 to 3, wherein the distillation residue or product mixture from linalool synthesis is used.

5. The process as claimed in claim 4, wherein the total liquid phase content of linalool and geraniol/nerol is less than 30% by weight.

6. The process as claimed in claim 4 or 5, wherein the product mixture comprises a total fraction of citronellol and isonerol of greater than 50% by weight.

7. The process as claimed in any of claims 1 to 6, wherein the hydrogenation is carried out using metal catalysts.

8. The process as claimed in claim 7, wherein the metal catalysts are selected from the metals of group VIIIA of the Periodic Table.

9. The process as claimed in claim 7 or 8, wherein the metal catalysts comprise Pd, Co or Ni.

10. The process as claimed in any of claims 7 to 9, wherein hydrogenation is carried out using Raney nickel.

11. The use of product mixtures or distillation residues from linalool, citronellal, citronellol or geraniol/nerol synthesis for the preparation of tetrahydrogeraniol.

12. The use of product mixtures or the distillation residues from linalool synthesis for the preparation of tetrahydrogeraniol using Ra catalysts.

## Revendications

1. Procédé de préparation de tétrahydrogéraniol, **caractérisé en ce que** les mélanges de produits et respectivement résidus de distillation obtenus lors de la synthèse de linalol, de citronellal, de citronellol ou de géraniol/nérol sont directement amenés à l'hydrogénation catalytique.

2. Procédé suivant la revendication 1, **caractérisé en ce que** les mélanges de produits et respectivement résidus de distillation contiennent plus d'un composé de la formule générale (I) : 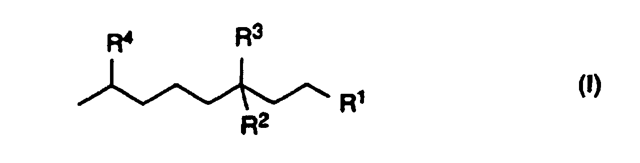 où
R¹ = OH, H, CH₃,
R², R³ = H, OH, CH₃,
R⁴ = H, CH₃,
et **en ce qu'**une à quatre doubles liaisons peuvent être contenues dans la molécule en position quelconque.

3. Procédé suivant l'une des revendications 1 et 2, **caractérisé en ce que** les mélanges de produits et respectivement résidus de distillation contiennent plus d'un composé choisi parmi le groupe du nérol, du géraniol, de l'isonérol 2, du citral, du citronellol, du 3,7-diméthyloctanal, de l'isonérol 1, du citronellal, du linalol.

4. Procédé suivant l'une des revendications 1 à 3, **caractérisé en ce que** le résidu de distillation ou respectivement le mélange de produits issu de la synthèse de linalol est utilisé.

5. Procédé suivant la revendication 4, **caractérisé en ce que** la teneur en linalol et en géraniol/nérol du fond de colonne est au total inférieure à 30% en poids.

6. Procédé suivant l'une des revendications 4 et 5, **caractérisé en ce que** le mélange de produits contient une fraction supérieure à 50% en poids de citronellol et d'isonérol au total.

7. Procédé suivant l'une des revendications 1 à 6, **caractérisé en ce que** l'hydrogénation a lieu sur des catalyseurs métalliques.

8. Procédé suivant la revendication 7, **caractérisé en ce que** les catalyseurs métalliques sont choisis parmi les métaux du groupe VIIIA du système périodique.

9. Procédé suivant la revendication 7 ou 8, **caractérisé en ce que** les catalyseurs métalliques contiennent Pd, Co ou Ni.

10. Procédé suivant l'une des revendications 7 à 9, **caractérisé en ce que** l'hydrogénation est effectuée sur du nickel de Raney.

11. Utilisation des mélanges de produits et respectivement des résidus de distillation de la synthèse de linalol, de citronellal, de citronellol ou de géraniol/nérol, pour la préparation de tétrahydrogéraniol.

12. Utilisation des mélanges de produits et respectivement des résidus de distillation de la synthèse de linalol, pour la préparation de tétrahydrogéraniol sur des catalyseurs de Ra.
